# EUROPEAN PATENT SPECIFICATION

(11) **EP 0 419 504 B1**
(45) Date of publication and mention of the grant of the patent: **26.01.1994**
(21) Application number: 89906057.8
(22) Date of filing: 10.05.1989
(51) Int. Cl.: C07K 7/40, A61K 37/26, C12P 21/02, C12N 15/00

(54) **INSULIN ANALOGUES**
INSULIN-ANALOGE
ANALOGUES D'INSULINE

(30) Priority: 11.05.1988 DK 2579/88
(43) Date of publication of application: 03.04.1991
(73) Proprietor: NOVO NORDISK A/S, 2880 Bagsvaerd (DK)
(72) Inventor: BRANGE, Jens, J rgen, Veilgaard, DK-2930 Klampenborg (DK); HAVELUND, Svend, DK-2880 Bagsvaerd (DK)
(74) Representative: Secher, Anne
(86) International application number: DK8900117
(87) International publication number: WO8910937

(56) References cited:
- SE-B- 451 461
- Chemiker-Zeitung, 100. Jahrgang (1976) Nr. 3 Rolf Geiger, "Chemie des Insulins", p. 111-129, see pages 120 and 126

## Description

### Technical Field

The present invention relates to novel insulin analogues being stabilized against chemical modifications and to novel insulin preparations containing such insulin analogues.

### Background of the invention

When insulin is handled at body - (or even room -) temperature the biological potency tends to decline as a function of time. Such loss of biological potency may be ascribed certain chemical modifications of insulin occurring during storage.

Like other proteins, insulin is not a stable entity but is liable to degradation by chemical reactions with molecules or ions in its vicinity, or to intra- and intermolecular transformations within the insulin molecules.

Hydrolytic transformation of insulin in acid solution with the liberation of ammonium ions and formation of deamidation products has been reported by Sundby (J.Biol.Chem. 237 (1962), 3406-4311) who demonstrated that monodesamido-(A21)-insulin is the prevailing derivative formed.

Jackson et al. (Diabetes 21 (1972), 235-245) compared acid and neutral solutions and showed that similar, but less pronounced deamidation can be observed in neutral regular insulin. Slow deamidation of insulin in neutral solution as well as in two different neutral suspensions was reported by Schlichtkrull et al. (Handbook of Experimental Pharmacology, New Series, Vol. XXXII/2, Springer Verlag, pp. 729-777).

At higher storage temperatures significant deamidation can also be observed in neutral solution in which the hydrolytic transformation has been shown to take place in the B-chain of insulin mainly at Asn(B3) (Brange et al., Diabetologia 25 (1983), 193 (abstract)). The rate of deamidation in neutral insulin preparations varies with the formulation.

Whereas desamido-insulin generated in acid medium is hydrolyzed in position A21 the products formed at neutral pH are deamidated in the B-chain partly by hydrolysis of Asn(B3), partly by an α-β aspartyl rearrangement of Asn(B3) with concomitant deamidation.

Formation of small amounts of covalent dimerization and polymerization products of insulin during storage of neutral preparations was reported by Schlichtkrull et al. (supra) and Brange et al., Diabetologia 27 (1984), 259. In neutral solutions this transformation varies with the formulation and the brand of insulin.

Insulin covalent dimerization is mainly due to a reaction of an N-terminal amino group with a carboxamide group of asparagine or glutamine.

Another part of dimerization is mainly due to a reaction of aldehyde with insulin amino groups forming a bridge between two insulin molecules.

It is the object of the present invention to provide novel insulin analogues which are substantially less susceptible to the above described chemical transformations and degradation.

The objectives of this invention are achieved with the novel human insulin analogues hereinafter described.

A large number of insulin analogues have been described in the past. Märki et al. (Hoppe-Seyler's Z.Physiol.Chem., 360 (1979), 1619-1632) describe synthesis of analogues of human insulin that differ from human insulin in the replacement of a single amino acid in positions 2, 5, 6, 7, 8, and 11 of the A-chain and 5, 7, 13: and 16 of the B-chain affording new insights into the intriguing structure activity relationship of insulin. Further studies modified the major receptor binding area in insulin (B(22)-B(26)) to investigate the impact of such mutation on the receptor binding activity. Stability against chemical degradation and transformation was, however, not discussed.

By "insulin analogues" as used herein is meant a compound having a molecular structure similar to that of insulin including the disulphide bridges between Cys)A7) and Cys(B7), and between Cys(A20) and Cys(B19) and an internal disulphide bridge between Cys(A6) and Cys(A11) and with insulin activity.

### Summary of the invention

In its broadest aspect the present invention provides insulin analogues wherein one or more asparagine residue (Asn) or glutamine residue (Gln) has been replaced by another naturally occurring amino acid residue.

The insulin analogues according to the present invention can be characterized in that Asn in position B3 is substituted by another naturally occurring amino acid residue and wherein further at least Gln in position A5 or A15 or Asn in position A18 or A21 is substituted by another naturally occurring amino acid residue and optionally also Gln in position B4 is replaced by another naturally occurring amino acid residue.

The substituents for Gln can in principle be any naturally occurring amino acid residue with no free amide group in the side chain. Preferred substituents are Glu, Asp, His, Ser, Thr, Val, Leu, Ile, Ala, Met, Trp, Tyr and Gly, Glu and Asp being most preferred.

The substituents for Asn can in principle be any naturally occurring amino acid residue, including Gln. Preferred substituents are Glu, Asp, His, Ser, Thr, Val, Leu, Ile, Ala, Gly, Met, Trp, Tyr and Gln, Glu and Asp being most preferred. Preferred substituents for Asn(B3) are Asp, Gln, Ser, Thr or Gly.

Although Asn will preferably be substituted with an amino acid residue containing no free amide group in the side chain, a stabilization against chemical degradation might in some instances be obtained by substituting Gln for Asn. The reason for this is that Asn is considered to be more susceptible than Gln to be deamidated, to form rearrangement products or to participate in dimerization reactions.

Replacement of Asn in B3 may be combined with replacement of Asn(A21) or Asn(A18). Also replacement of Asn in B3 may be combined with replacement of Asn in A18 and A21 or Gln in A15 and Asn in A18.

The present invention is not contemplated to be related to stabilization of native insulins such as human, pork or beef insulin, only. In the recent years a number of insulin analogues have been developed in which certain amino acid residues of human insulin have been replaced by other amino acid residues.

In European patent application No. 214826 rapid acting insulin analogues are described in which one or more of the amino acid residues in the positions A8, A9, A10, A13, A21, B1, B2, B5, B9, B10, B12, B14, B16, B17, B18, B20, B26, B27 and B28 of human insulin are replaced by another amino acid residue. Preferred substituents are Glu and Asp. As these insulin analogues have retained Asn and Gln in positions A18 and B3 for A5, A15 and B4, respectively, they may as well be stabilized against chemical degradation according to the present invention.

Examples of such rapid acting insulin analogues being stabilized against chemical degradation are such containing an Asp or Glu in position B9, B10, B27 and/or B28.

Another group of known insulin analogues are described in European patent application No. 254,516. In these insulin analogues a basic amino acid residue has been substituted in B27 position and/or a neutral amino acid residue has been inserted in the A4, A17, B13 and/or B21 position. Furthermore Asn(A21) may be substituted by another amino acid residue in order to increase the stability of insulin solution containing the protracted insulin analogues at acid pH. The present invention, however, goes further than what is described in EP No. 254,516 suggesting substitutions of Gln and/or Asn in position A5, A15, A18, B3 and B4 as well.

The present invention is also contemplated to comprise certain derivations or further substitutions of the insulin analogues provided that such derivations or substitutions have no substantial impact on the aimed goal of the invention. It is accordingly possible to derivate one or more of the functional groups in the amino acid residues. For instance, intended to be within the scope of this invention would be per se known conversion of acid groups in the insulin molecule into ester or amide groups and conversion of alcohol groups into alkoxy groups or vice versa.

Also intended to be within the scope of this invention is addition or removal of a few amino acid residues at the N- or C-terminal ends of the A- and B-chains provided that this has no significant impact on the overall properties of the insulin analogues.

Such modifications at the ends of the A- and B-polypeptide chains may be exercised in vitro on the insulin analogues according to the present invention or by means of recombinant DNA-technology, as will be apparent for the person skilled in the art.

The novel insulin analogues according to the present invention may be prepared by altering the proinsulin gene through replacement of codon(s) at the appropriate site in the native human proinsulin gene by codon(s) encoding the desired amino acid residue substitute(s) or by synthesizing the whole DNA-sequence encoding the desired insulin analogue. The gene encoding the desired insulin analogue is then inserted into a suitable expression vector which when transferred to a suitable host organism, e.g. E. coli, Bacillus or yeast, generates the desired product. The expressed product is then isolated from the cells or the culture broth depending on whether the expressed product is secreted from the cells or not.

The novel insulin analogues may also be prepared by chemical synthesis by methods analogue to the method described by Märki et al. (Hoppe-Seyler's Z. Physiol.Chem., 360 (1979), 1619-1632). They may also be formed from separately in vitro prepared A- and B-chains containing the appropriate amino acid residue substitutions, whereupon the modified A- and B-chains are linked together by establishing disulphide bridges according to known methods (e.g. Chance et al., In: Rick DH, Gross E (eds) Peptides: Synthesis - Structure - Function. Proceedings of the seventh American peptide symposium, Illinois, pp. 721-728).

The insulin analogues may furthermore be prepared by a method analogue to the method described in EP patent application No. 0163529A, the disclosure of which is incorporated by reference hereinto. By such a method an insulin precursor of human insulin wherein Lys^{B29} is connected to Gly^{A21} by means of either a peptide bond or a peptide chain of varying length with correctly positioned disulphide bridges is expressed and secreted by yeast and then converted into human insulin by the so-called transpeptidation reaction.

Accordingly the present insulin analogues may be prepared by inserting a DNA-sequence encoding a precursor of the insulin analogue in question into a suitable yeast expression vehicle which when transferred to yeast is capable of expressing and secreting the precursor of the insulin analogue in which Lys^{B29} is connected to Gly^{A21} by a peptide bond or a peptide chain with the formula I

-Rₙ-R¹- (I)

wherein R is a peptide chain with n amino acid residues, n is an integer from 0 to 33 and R¹ is Lys or Arg when culturing the transformed yeast strain in a suitable nutrient medium. The precursor is then recovered from the culture broth and reacted with an amino compound with the formula II

Q-OR'' (II)

wherein Q is the amino acid residue which is to be inserted in the B30 position, preferably Thr, and R'' is a carboxy protecting group (e.g. methyl or tert-butyl), using trypsin or trypsin-like enzyme as a catalyst in a mixture of water and organic solvents analogously as described in US patent specification No. 4,343,898 (the disclosure of which is incorporated by reference hereinto) whereupon the carboxy protecting group is removed and the insulin analogue is isolated from the reaction mixture.

The insulin analogues may also be prepared by a method analogue to the method described in EP patent application No. 86302133.3 the disclosure of which is incorporated by reference hereinto. By this method insulin precursors of the type having a bridge between the A- and B-chain consisting of a single pair of basic amino acid (Lys, Arg) are made in yeast and then converted into insulin by an enzymatic conversion.

The present insulin analogues may be used for the preparation of novel insulin preparations with insulin activity to be substituted for human or porcine insulin in the insulin preparations heretofore known to the art. Such novel insulin preparations contain the insulin analogues according to the present invention or a pharmaceutically acceptable salt thereof in aqueous solution or suspension, preferably at neutral pH. The aqueous medium is made isotonic, for example with sodium chloride, sodium acetate or glycerol. Furthermore, the aqueous medium may contain zinc ions, buffers such as acetate and citrate and preservatives such as m-cresol, methylparaben or phenol. the pH value of the preparation is adjusted to the desired value. The insulin preparation is made sterile by sterile filtration.

The insulin preparations of this invention can be used similarly to the use of the known insulin preparations.

### Terminology

The abbreviations used for the amino acids are those stated in J.Biol.Chem. 243 (1968), 3558. The amino acids are in the L configuration. Unless otherwise indicated, the species of insulins stated herein is human.

As used in the following text B(1-29) means a shortened B-chain of human insulin from B(1)Phe to B(29)lys and A(1-21) means the A-chain of human insulin.

The substitution(s) made in the human insulin molecule according to the practice of the invention is (are) indicated with a prefix referenced to human insulin. As an example Glu(B3) human insulin means a human insulin analogue wherein Glu has been substituted for Asn in position 3 in the B-chain. Glu(B3),B(1-29)-Ala-Ala-Lys-A(1-21) human insulin means a precursor for the insulin analogue wherein Glu has been substituted for Asn in position 3 in the shortened B-chain (see above) and wherein the B(1-29)-chain and the A-chain (A(1-21)) are connected by the peptide sequence Ala-Ala-Lys. Unless otherwise stated it is to be understood that the B(1-29) chain and A(1-21) chain are connected by disulphide bridges between A(7)Cys and B(7)Cys and between A(20)Cys and B(19)Cys, respectively, as in human insulin and that the A-chain contains the internal disulphide bridge between A(6)Cys and A(11)Cys.

### Detailed description

Genes encoding the precursors of the insulin analogue can be prepared by modification of genes encoding the corresponding human insulin precursors by site specific mutagenesis to insert or substitute with codons encoding the desired mutation. A DNA-sequence encoding the precursor of the insulin analogue may also be made by enzymatic synthesis form oligonucleotides corresponding in whole or part to the insulin analogue precursor gene.

DNA-sequences containing a gene with the desired mutation are then combined with a suitable promoter sequence, e.g. fragments coding for the TPI promoter (TPIp) (T. Alber and G. Kawasaki,. Nucleotide Sequence of the triose Phosphate Isomerase Gene of Saccharomyces cerevisiae. J.Mol.Applied Genet. 1 (1982), 419-434), a suitable leader sequence and possible transcription termination sequence, e.g. from TPI of S. cerevisiae (TPI_{T}). These fragments provide sequences to ensure a high rate of transcription for the precursor encoding gene and also provide a presequence which can effect the localization of precursor into the secretory pathway and its eventual excretion into the growth medium. The expression units are furthermore provided with a yeast origin of replication, for instance the 2µ origin, and a selectable marker, for instance LEU 2.

For the purpose of this invention we prepared the genes encoding the insulin analogue precursor in question by ligation of 10 oligonucleotides followed by insertion of the gene into a yeast expression plasmid as described by L. Thim et al., Proc.Natl.Acad.Sci. USA 83 (1986), 6766-6770, and J. Markussen et al., Protein Engineering 1 (1987), 215-223.

### Example 1

### Production of Glu(A15), Asp(A18), Asp(B3) human insulin

A synthetic gene for the precursor Asp(B3)B(1-29)-Ala,Ala-Lys-Glu(A15),Asp(A18),A(1-21) was constructed from 10 oligonucleotides by ligation. The oligonucleotides were synthesized on an automatic DNA synthesizer using phosphoramidite chemistry on a controlled pore glass support (Beaucage, S.L. and Caruthers, M.H. Tetrahydron Letters 22 (1981), 1859-1869). The synthetic gene was as follows: 
B1  2   3   4   5   6   7   8   9  10  11  12  13  14  15  16  17  18  19
 F   V   D   Q   H   L   C   G   S   H   L   V   E   A   L   Y   L   V   C
HinfI SalI              NlaIV           HindIII RsaI

ATTCGTCGACCAACACTTGTGCGGTTCCCACTTGGTTGAAGCTTTGTACTTGGTTTGC-
    GCAGCTGGTTGTGAACACGCCAAGGGTGAACCAACTTCGAAACATGAACCAAACG-

20  21  22  23  24  25  26  27  28  29          A1  2   3   4   5   6
 G   E   R   G   F   F   Y   T   P   K AlaAlaLys G   I   V   E   Q   C
 HphI           MboII     MstII,DdeI   DdeI         TagI

GGTGAAAGAGGTTTCTTCTACACTCCTAAGGCTGCTAAGGGTATTGTCGAACAATGC-
CCACTTTCTCCAAAGAAGATGTGAGGATTCCGACGATTCCCATAACAGCTTGTTACG-

 7   8   9  10  11  12  13  14  15  16  17  18  19  20  21
 C   T   S   I   C   S   L   Y   E   L   E   D   Y   C   N
 RsaI                   RsaI (-PvuII)                HgaI

TGTACCTCCATCTGCTCCTTGTACGAATTGGAAGACTACTGCAACTAGACGCAGCC-
ACATGGAGGTAGACGAGGAACATGCTTAACCTTCTGATGACGTTGATCTGCGTCGG-

      XbaI

CGCAGGCT
GCGTCCGAGATC
Letters and numbers above the DNA-sequence indicate the corresponding amino acid residue (by means of the one letter abbreviation) and its position, respectively, in the B- and in the A-chain. The sequence AlaAlaLys is the bridge between the A- and B-chain. Also shown are the endonuclease restriction sites in the synthetic gene.

The synthetic gene was ligated to a 330 bp EcoRI-HgaI fragment from plasmid pKFN9 coding for MFαl signal and leader sequence(1-85) and to the large EcoRI-XbaI fragment from pUC19. The construction of pKFN9 containing a HgaI site immediately after the MFαl leader sequence is described in EP patent application No. 0214826.

The ligation mixture was used to transform a competent E. coli strain (r⁻, m⁺) selecting for ampicillin resistance. Sequencing of a ³²P-XbaI-EcoRI fragment (Maxam, A. and Gilbert, W., Methods Enzymol. 65 (1980), 499-560) showed that plasmids from the resulting colonies contained the correct DNA-sequence.

One correct plasmid was selected for further use and cut with EcoRI and XbaI. The EcoRI-XbaI fragment was ligated to a 9.5 kb NcoI-XbaI fragment from pMT636 and a 1.4 kb NcoI-EcoRI fragment from pMT636. The construction of plasmid pMT636 is described in WO patent application No. 89/01968. It contains the Schizo. pombe TPI gene (POT), the S. cerevisiae triosephosphate isomerease promoter and terminator, TPI_{P} and TPI_{T} (Alber, T. and Kawasaki, G., J.Mol.Appl.Gen. 1 (1982), 419-434).

The resulting plasmid encodes for the following sequence
TPI_{P}-MFα1-signal-leader(1-85)-precursor gene-TPI_{T}
where MFα1 is the S. cerevisiae MFα1 coding sequence (Kurjan, J. and Herskowits, I., Cell 30 (1982), 933-943) and signal-leader(1-85) means that the sequence contains the first 85 amino acid residues of the MFa1 signal-leader sequence.

An S. cerevisiae (E2-7B XE11-36 a/α, Δtpi Δtpi, pep 4-3/pep 4-3) was grown on YPGaL (1% Bacto yeast extract, 2% Bacto peptone, 2% galactose, 1% lactate) to an O.D. at 600 nm of 0.6.

100 ml of culture was harvested by centrifugation, washed with 10 ml of water, recentrifuged and resuspended in 10 ml of a solution containing 1.2 M sorbitol, 25 mM Na₂EDTA pH = 8.0, and 6.7 mg/ml dithiotreitol. The suspension was incubated at 30°C for 15 minutes, centrifuged and the cells resuspended in 10 ml of a solution containing 1.2 M sorbitol, 10 mM Na₂EDTA, 0.1 M sodium citrate, pH = 5.8, and 2 mg Novozym^{(R)} 234. The suspension was incubated at 30°C for 30 minutes, the cells collected by centrifugation, washed in 10 ml of 1.2 M sorbitol and 10 ml of CAS (1.2 M sorbitol, 10 mM CaCl₂, 10 mM Tris-HCl (Tris = Tris(hydroxymethyl)aminomethan) pH = 7.5) and resuspended in 2 ml of CAS. For transformation 0.1 ml of CAS-resuspended cells were mixed with approximately 1 µg of the above described plasmid and left at room temperature for 15 minutes. 1 ml of (20% polyethylenglycol 4000, 10 mM CaCl₂, 10 mM Tris-HCl, pH = 7.5) was added and the mixture left for further 30 minutes at room temperature. The mixture was centrifuged and the pellet resuspended in 0.1 ml of SOS (1.2 M sorbitol, 33% v/v YPD, 6.7 mM CaCl₂, 14 µg/ml leucine) and incubated at 30°C for 2 hours. The suspension was then centrifuged and the pellet resuspended in 0.5 ml of 1.2 M sorbitol. Then, 6 ml of top agar (the SC medium of Sherman et al., (Methods in Yeast Genetics, Cold Spring Harbor Laboratory, 1981) containing 1.2 M sorbitol plus 2.5% agar) at 52°C was added and the suspension poured on top of plates containing the same agar-solidified, sorbitol containing medium. Transformant colonies were picked after 3 days at 30°C, resisolated and used to start liquid cultures.

Transformant strains were grown on YPD medium (1% yeast extract, 2% peptone (from Difco laboratories), and 2% glucose) in a 1500 liter tank at 30°C. The expressed product was isolated from the culture broth. The yield was 40.3 mg/liter.

### Transpeptidation

0.2 mole (47.1 g) Thr-Met, HOAC was dissolved in DMF to give 100 ml solution, 50 ml 76.5% v/v DMF in water was added and 10 g of crude Asp(B3),B(1-29)-Ala-Ala-Lys-Glu(A15),Asp(A18)A(1-21) human insulin was dissolved in the mixture, which was thermostated at 12°C. Then 1 g of trypsin in 25 ml 0.05 M calcium acetate was added and after 24 hours at 12°C the mixture was added to 2 liter of acetone and the precipitated peptides were isolated by centrifugation and dried in vacuo. The Glu(A15),Asp(A18), Asp(B3),B30Thr-OMe human insulin was purified on a preparative HPLC column with silica-C18 as column material.

The Glu(A15),Asp(A18),Asp(B3),B30Thr-OMe human insulin was dispersed in water to 1% (w/v) and was dissolved by addition of 1 N sodium hydroxide to a pH value of 10.0. The pH value was kept constant at 10.0 for 24 hours at 25°C. The Glu(A15),Asp(A18),Asp(B3) human insulin formed was precipitated by addition of sodium chloride to about 8% (w/v), sodium acetate trihydrate to about 1.4% (w/v), and zinc acetate dihydrate to about 0.01% (w/v) followed by addition of 1 N hydrochloric acid to pH 5.5. The precipitate was isolated by centrifugation and purified by anion exchange chromotography and desalted by gel filtration. Yield: 2.40 g of Glu(A15),Asp(A18),Asp(B3) human insulin.

### Example 2

### Preparation of Glu(A18),Glu(B3),Asp(B10) human insulin

A synthetic gene encoding a precursor Glu(B3),Asp(B10)-Ala-Ala-Lys-Glu(A18)A(1-21) was prepared as described in Example 1. The gene had the following sequence: 
B1  2  3  4  5  6  7  8  9  10  11  12  13  14  15  16  17  18  19
F  V  E  Q  H  L  C  G  S  D  L  V  E  A  L  Y  L  V  C
HinfI                    NlaIV              HindIII  RsaI

ATTCGTTGAACAACACTTGTGCGGTTCCGACTTGGTTGAAGCTTTGTACTTGGTTTGC-
    GCAACTTGTTGTGAACACGCCAAGGCTGAACCAACTTCGAAACATGAACCAAACG-

20  21  22  23  24  25  26  27  28  29              A1  2  3  4  5  6
G  E  R  G  F  F  Y  T  P  K  AlaAlaLys  G  I  V  E  Q  C
HphI          MboII      MstII,DdeI    DdeI          TagI

GGTGAAAGAGGTTTCTTCTACACTCCTAAGGCTGCTAAGGGTATTGTCGAACAATGC-
CCACTTTCTCCAAAGAAGATGTGAGGATTCCGACGATTCCCATAACAGCTTGTTACG-

7  8  9  10  11  12  13  14  15  16  17  18  19  20  21
C  T  S  I  C  S  L  Y  Q  L  E  E  Y  C  N
RsaI                    RsaI (-PvuII)  ScaI          HgaI

TGTACCTCCATCTGCTCCTTGTACCAATTGGAAGAGTACTGCAACTAGACGCAGCC-
ACATGGAGGTAGACGAGGAACATGGTTAACCTTCTCATGACGTTGATCTGCGTCGG-

XbaI

CGCAGGCT
GCGTCCGAGATC
Letters and numbers above the DNA-sequence indicate the corresponding amino acid residue (by means of the one letter abbreviation) and its position, respectively, in the B- and in the A-chain. The sequence AlaAlaLys is the bridge between the A- and B-chain. Also shown are the endonuclease restriction sites in the synthetic gene.

The synthetic gene was inserted into a yeast transformant plasmid as described in Example 1. Transformation of yeast and cultivation of the transformed yeast strain was conducted as described in Example 1. Yield of the precursor was 87.3 mg/liter. The precursor was transpeptidized and converted into the above endproduct by the same procedure as in Example 1. Yield of Glu(A18),Glu(B3),Asp(B10) human insulin was 17.73 g.

### Example 3

### Preparation of Gly(A21), Gln(B3) human insulin

A synthetic gene for the precursor Gln(B3)B(1-29)-Ala-Ala-Lys-Gly(A21)A(1-21) was constructed as described in Example 1. The synthetic gene had the following sequence: 
B1  2  3  4  5  6  7  8  9 10 11 12 13 14 15 16 17 18 19
 F  V  Q  Q  H  L  C  G  S  H  L  V  E  A  L  Y  L  V  C

HinfI                    NlaIV              HindIII RsaI

ATTCGTTCAACAACACTTGTGCGGTTCCCACTTGGTTGAAGCTTTGTACTTGGTTTGC-
   GCAAGTTGTTGTGAACACGCCAAGGGTGAACCAACTTCGAAACATGAACCAAACG-

20 21 22 23 24 25 26 27 28 29          A1  2  3  4  5  6
 G  E  R  G  F  F  Y  T  P  K AlaAlaLys G  I  V  E  Q  C
 HphI        MboII    MstII,DdeI   DdeI        TagI

GGTGAAAGAGGTTTCTTCTACACTCCTAAGGCTGCTAAGGGTATTGTCGAACAATGC-
CCACTTTCTCCAAAGAAGATGTGAGGATTCCGACGATTCCCATAACAGCTTGTTACG-

 7  8  9 10 11 12 13 14 15 16 17 18 19 20 21
 C  T  S  I  C  S  L  Y  Q  L  E  N  Y  C  G
 RsaI                RsaI (-PvuII)              XbaI

TGTACCTCCATCTGCTCCTTGTACCAATTGGAAAACTACTGCGGTTAAT
ACATGGAGGTAGACGAGGAACATGGTTAACCTTTTGATGACGCCAATTAGATC
Letters and numbers above the DNA-sequence indicate the corresponding amino acid residue (by means of the one letter abbreviation) and its position, respectively, in the B- and in the A-chain. The sequence AlaAlaLys is the bridge between the A- and B-chain. Also shown are the endonuclease restriction sites in the synthetic gene.

The synthetic gene was inserted into a yeast transformant plasmid as described in Example 1. Transformation of yeast and cultivation of the transformed yeast strain was conducted as described in Example 1. Yield of the precursor was 19.6 mg/liter. The precursor was transpeptidized and converted into the above endproduct by the same procedure as in Example 1. Yield of Gly(A21),Gln(B3) human insulin was 0.31 g.

### Example 4

### Preparation of His(A18) ,Ser(A21) ,Thr(B3) human insulin

A synthetic gene for the precursor Thr(B3)B(1-29)-Ala-Ala-Lys-His(A18),Ser(A21)A(1-21) was constructed as described in Example 1. The synthetic gene had the following sequence: 
B1  2  3  4  5  6  7  8  9 10 11 12 13 14 15 16 17 18 19
 F  V  T  Q  H  L  C  G  S  H  L  V  E  A  L  Y  L  V  C
HinfI MaeIII        NlaIV        HindIII RsaI

ATTCGTTACCCAACACTTGTGCGGTTCCCACTTGGTTGAAGCTTTGTACTTGGTTTGC-
   GCAATGGGTTGTGAACACGCCAAGGGTGAACCAACTTCGAAACATGAACCAAACG-

20 21 22 23 24 25 26 27 28 29          A1  2  3  4  5  6
 G  E  R  G  F  F  Y  T  P  K AlaAlaLys G  I  V  E  Q  C
 HphI        MboII    MstII,DdeI   DdeI        TagI

GGTGAAAGAGGTTTCTTCTACACTCCTAAGGCTGCTAAGGGTATTGTCGAACAATGC-
CCACTTTCTCCAAAGAAGATGTGAGGATTCCGACGATTCCCATAACAGCTTGTTACG-

 7  8  9 10 11 12 13 14 15 16 17 18 19 20 21
 C  T  S  I  C  S  L  Y  Q  L  E  H  Y  C  T
 RsaI              RsaI (-PvuII)      PstI   XbaI

TGTACCTCCATCTGCTCCTTGTACCAATTGGAACACTACTGCAGTTAAT
ACATGGAGGTAGACGAGGAACATGGTTAACCTTGTGATGACGTCAATTAGATC
Letters and numbers above the DNA-sequence indicate the corresponding amino acid residue (by means of the one letter abbreviation) and its position, respectively, in the B- and in the A-chain. The sequence AlaAlaLys is the bridge between the A- and B-chain. Also shown are the endonuclease restriction sites in the synthetic gene.

The synthetic gene was inserted into a yeast transformant plasmid as described in Example 1. Transformation of yeast and cultivation of the transformed yeast strain was conducted as described in Example 1. Yield of the precursor was 7.3 mg/liter. The precursor was transpeptidized and converted into the above endproduct by the same procedure as in Example 1. Yield of His(A18),Ser(A21),Thr(B3) human insulin was 0.79 g.

Neutral solutions of the above insulin analogues containing phenol and glycerol were tested for stability after storage for four weeks at 37°C. As reference was used a zinc free neutral solution of human insulin and a rapid acting insulin Asp(B10) human insulin. Percent of desamido products were determined by HPLC. The results appear from the following table.

**Table 1**

| Tested compound | % desamido-insulin |
|---|---|
| Glu(A15),Asp(A18),Asp(B3) human insulin | 0.7 |
| Gly(A21),Gln(B3) human insulin | 0.3 |
| His(A18),Ser(A21),Thr(B3) human insulin | 0.7 |
| Human insulin, zinc free (reference) | 8.5 |
| Glu(A18),Gln(B3),Asp(B10) human insulin | 0.7 |
| Asp(B10) human insulin (reference) | 3.2 |

It appears from the results in Table 1 that the insulin analogues according to the present invention are markedly less deamidated than the reference compounds and therefore more stable at storage.

The novel insulin was tested for in vitro insulin activity with human insulin as standard by a free fat cell assay (FFC) as described by A.J. Moody et al., Hormone Metab.Res. 6 (1974), 12-16 using mouse adipocytes. The results are shown in the following table.

**Table 2**

| Tested compound | FFC % of human insulin |
|---|---|
| Glu(A15),Asp(A18),Asp(B3) human insulin | 58 |
| Glu(A18),Glu(B3),Asp(B10) human insulin | 67 |
| Gly(A21),Gln(B3) human insulin | 62.7 |
| His(A18),Ser(A21),Thr(B3) human insulin | 33.2 |

In vitro biological activity within the above magnitude will correspond to an in vivo biological activity of the same magnitude as that of human insulin (U. Ribel et al., Diabetes Research and Clinical Practice, Supp. 1 to vol 5, pos. 003-3, 1988).

## Claims

1. Human insulin analogues wherein Asn in position B3 is substituted by another naturally occurring amino acid residue and wherein further at least Gln in position A5 or A15 or Asn in position A18 or A21 is substituted by another naturally occurring amino acid residue and optionally also Gln in position B4 is replaced by another naturally occurring amino acid residue.

2. Human insulin analogues according to claim 1, characterized in that Asn in position B3 and A18 is replaced by another naturally occurring amino acid residue.

3. Human insulin analogues according to claim 1, characterized in that Asn in B3 and A21 is replaced by another naturally occurring amino acid residue.

4. Human insulin analogues according to claim 1, characterized in that Asn in position B3 and A21 and Gln in position A15 is replaced by another naturally occurring amino acid residue.

5. Human insulin analogues according to the previous claims, characterized in that the amino acid residue replacement in positions A18, A21 and B3 is selected from the group consisting of Glu, Asp, His, Ser, Thr, Val, Leu, Ile, Ala, Met, Trp, Tyr, Gly and Gln, and the amino acid residue replacement in position A5, A15 and B4 is selected from the group consisting of Glu, Asp, His, Ser, Thr, Val, Leu, Ile, Ala, Met, Trp, Tyr and Gly.

6. Human insulin analogues according to claim 1, characterized in that the amino acid residue in position B3 is Asp, Gln, Ser, Thr or Gly.

7. Insulin preparations with insulin activity, characterized in that they contain an insulin analogue according to any of the previous claims or a pharmaceutically acceptable salt thereof.

8. A process for the preparation of human insulin according to claim 1, characterized in that a yeast strain transformed with a replicable expression vehicle comprising a DNA-sequence encoding a precursor of the insulin analogue in which Lys^{B29} is connected to Gly^{A1} by a peptide bond or a peptide chain with the formula I
-Rₙ-R^{¹}- (I)
wherein R is a peptide chain with n amino acid residues, n is an integer from 0 to 33 and R¹ is Lys or Arg, is cultured in a suitable nutrient medium, the precursor is recovered from the culture broth and reacted with an amino compound with the formula II
Thr-OR'' (II)
wherein R'' is a carboxy protecting group, using trypsin or a trypsin-like enzyme as a catalyst in a mixture of water and organic solvents whereafter the carboxy protecting group is removed by hydrolysis.

## Patentansprüche

1. Humaninsulin-Analoge, in denen Asn in Position B3 durch einen anderen natürlich auftretenden Aminosäurerest ersetzt ist und in denen außerdem wenigstens Gln in Position A5 oder A15 oder Asn in Position A18 oder A21 durch einen anderen natürlich auftretenden Aminosäurerest ersetzt ist und fakultativ auch Gln in Position B4 durch einen anderen natürlichen auftretenden Aminosäurerest ersetzt ist.

2. Humaninsulin-Analoge nach Anspruch 1, dadurch gekennzeichnet, daß Asn in Position B3 und A18 durch einen anderen natürlich auftretenden Aminosäurerest ersetzt ist.

3. Humaninsulin-Analoge nach Anspruch 1, dadurch gekennzeichnet, daß Asn in B3 und A21 durch einen anderen natürlich auftretenden Aminosäurerest ersetzt ist.

4. Humaninsulin-Analoge nach Anspruch 1, dadurch gekennzeichnet, daß Asn in Position B3 und A21 und Gln in Position A15 durch einen anderen natürlich auftretenden Aminosäurerest ersetzt ist.

5. Humaninsulin-Analoge nach den vorangehenden Ansprüchen, dadurch gekennzeichnet, daß der Aminosäurerest-Ersatz in den Positionen A18, A21 und B3 ausgewählt ist aus der Gruppe, die aus Glu, Asp, His, Ser, Thr, Val, Leu, Ile, Ala, Met, Trp, Tyr, Gly und Gln besteht, und der Aminosäurerest-Ersatz in Position A5, A15 und B4 ausgewählt ist aus der Gruppe, die aus Glu, Asp, His, Ser, Thr, Val, Leu, Ile, Ala, Met, Trp, Tyr und Gly besteht.

6. Humaninsulin-Analoge nach Anspruch 1, dadurch gekennzeichnet, daß der Aminosäurerest in Position B3 Asp, Gln, Ser, Thr oder Gly ist.

7. Insulin-Zubereitungen mit Insulinaktivität, dadurch gekennzeichnet, daß sie ein Insulin-Analog nach einem der vorangehenden Ansprüche oder ein pharmazeutisch annehmbares Salz desselben enthalten.

8. Verfahren zur Herstellung von Humaninsulin nach Anspruch 1, dadurch gekennzeichnet, daß ein Hefestamm, der mit einem replizierbaren Expressionsvehikel transformiert ist, das eine DNA-Sequenz umfaßt, die einen Vorläufer des Insulin-Analogs kodiert, in dem Lys^{B29} mit Gly^{A1} verknüpft ist durch eine Peptidbindung oder eine Peptidkette mit der Formel I
-Rₙ-R¹-, (I)
in der R eine Peptidkette mit n Aminosäureresten ist, n eine ganze Zahl von 0 bis 33 ist und R¹ Lys oder Arg ist, in einem geeigneten Nährstoffmedium kultiviert wird, der Vorläufer aus der Kulturbrühe gewonnen und mit einer Aminoverbindung mit der Formel II
Thr-OR'',(II)
in der R'' ein Carboxy-Schutzgruppe ist, unter Verwendung von Trypsin oder einem trypsinähnlichen Enzym als einem Katalysator in einer Mischung aus Wasser und organischen Lösungsmitteln zur Reaktion gebracht wird, woraufhin die Carboxy-Schutzgruppe durch Hydrolyse entfernt wird.

## Revendications

1. Analogues d'insuline humaine dans lesquels Asn en position B3 est remplacé par un autre résidu d'aminoacide naturel et dans lesquels au moins Gln en position A5 ou A15 ou Asn en position A18 ou A21 est remplacé par un autre résidu d'aminoacide naturel et éventuellement Gln en position B4 est aussi remplacé par un autre résidu d'aminoacide naturel.

2. Analogues d'insuline humaine selon la revendication 1, caractérisés en ce que Asn en position B3 et A18 est remplacé par un autre résidu d'aminoacide naturel.

3. Analogues d'insuline humaine selon la revendication 1, caractérisés en ce que Asn en B3 et A21 est remplacé par un autre résidu d'aminoacide naturel.

4. Analogues d'insuline humaine selon la revendication 1, caractérisés en ce que Asn en B3 et A21 et Gln en position A15 sont remplacés par un autre résidu d'aminoacide naturel.

5. Analogues d'insuline humaine selon les revendications précédentes, caractérisés en ce que le résidu d'aminoacide de remplacement en position A18, A21 et B3 est choisi dans le groupe constitué par Glu, Asp, His, Ser, Thr, Val, Leu, Ile, Ala, Met, Trp, Tyr, Gly et Gln, et le résidu d'aminoacide de remplacement en position A5, A15 et B4 est choisi dans le groupe constitué par Glu, Asp, His, Ser, Thr, Val, Leu, Ile, Ala, Met, Trp, Tyr et Gly.

6. Analogues d'insuline humaine selon la revendication 1, caractérisés en ce que le résidu d'aminoacide en position B3 est Asp, Gln, Ser, Thr ou Gly.

7. Préparations d'insuline à activité d'insuline, caractérisées en ce qu'elles contiennent un analogue d'insuline selon l'une quelconque des revendications précédentes ou un de leurs sels pharmaceutiquement acceptables.

8. Procédé de préparation d'insuline humaine selon la revendication 1, caractérisé en ce que l'on cultive dans un milieu nutritif convenable une souche de levure transformée avec un véhicule d'expression réplicable comprenant une séquence d'ADN codant pour un précurseur de l'analogue d'insuline dans lequel Lys^{B29} est relié à Gly^{A1} par une liaison peptidique ou une chaîne peptidique de formule I
-Rₙ-R¹- (I)
dans laquelle R est une chaîne peptidique de n résidus d'aminoacides, n est un entier de 0 à 33 et R¹ est Lys ou Arg, on récupère le précurseur à partir du bouillon de culture et on le fait réagir avec un composé amino de formule II
Thr-OR'' (II)
dans laquelle R'' est un groupe protecteur de carboxy, avec de la trypsine ou une enzyme analogue à la trypsine comme catalyseur dans un mélange d'eau et de solvants organiques, après quoi on élimine le groupe protecteur de carboxy par hydrolyse.
